# EUROPEAN PATENT APPLICATION

(11) **EP 3 611 264 A1**
(43) Date of publication of application: **19.02.2020**
(21) Application number: 19190227.9
(22) Date of filing: 06.08.2019
(51) Int. Cl.: C12N 15/113, A61P 37/00, A61P 29/00

(54) **PHARMACEUTICAL COMPOSITION FOR TREATING CHRONIC INFLAMMATORY DISEASES COMPRISING MIR-369-3P AS ACTIVE INGREDIENT**

(30) Priority: 08.08.2018 IT 201800007954
(71) Applicant: Ente Ospedaliero Specializzato In Gastroenterologia "Saverio De Bellis", 70013 Castellana Grotte (BA) (IT)
(72) Inventor: Serino, Grazia, 76123 Andria (IT)
(74) Representative: De Tullio, Michele Elio

(57) **Abstract**

The present invention relates to a pharmaceutical composition comprising one or more microRNAs, at least one vehicle and/or diluent and/or excipient pharmaceutically acceptable, characterized by comprising miR-369-3p as active ingredient. This composition is indicated for use in the treatment of chronic inflammatory diseases, in particular for the treatment of inflammatory bowel diseases.

## Description

The present invention relates in general to the pharmaceutical field which used microRNAs for therapeutic and diagnostic purposes. More in detail, the present invention relates to a pharmaceutical composition containing one or more microRNA and in particular miR-369-3p.

This pharmaceutical composition, based on miR-369-3p as active ingredient, shows a significant anti-inflammatory activity; said anti-inflammatory activity is mediated by dendritic cells that are cell-mediated immunity cells.

More specifically, the anti-inflammatory activity is related to a significant reduction of some chemicals mediators of the inflammatory response, as in particular C/EBP-β, TNF-α e IL-6.

In particular, this pharmaceutical composition is indicated for the treatment of the chronic inflammatory disorders and more specifically for the inflammatory gastrointestinal disease.

### Background art

MicroRNAs (miRNAs) are a class of small, endogenous RNAs with a length of 21-25 nucleotides. They play an important regulatory role in inhibiting the translation of specific mRNAs, acting as important regulators of the various processes including cell proliferation, apoptosis, fat metabolism, neuronal patterning, hematopoietic differentiation and immunity (He and Hannon, 2004). Accumulating data on their action in regulating immune cell function and inflammation, highlight miRNAs as a new class of biomarkers and drug targets (Sonkoly and Pivarcsi, 2009). In particular, several miRNAs involved in the inflammatory response have been identified, namely miR-155, miR-146a and miR-132 (O' Connell et al., 2009; Taganov et al., 2006; Nahid et al., 2013).

Dendritic cells (DCs) are the most potent antigen-presenting cells and play an important role in the crosstalk between the innate and the adaptive immune response (Steinman and Banchereau, 2007; Janeway and Medzhitov, 2002). DCs constantly supervise all body tissues in their immature form (iDCs) and rapidly respond when exposed to pattern recognition receptors (PRRs) like toll-like receptors (TLRs), or intracellular PRRs like NOD-like receptors (NLRs) becoming mature DCs (mDCs) (Mellman, 2013). Among all the PRRs, the lipopolysaccharide (LPS), the TLR4 ligand, is among the best-known maturational stimuli of DCs (Mellman, 2013). Mature DCs possess limited endocytosis, but exceptional T cells stimulating capacity. This is due to the ability to present previously encountered antigens on their major histocompatibility complex (MHC) class II (Inaba et al., 2000). To better fulfill their antigens presenting cell mission, the mDCs express higher levels of costimulatory molecules (CD40, CD80 and CD86) (Caux et al., 1994), undergo a chemokine receptors switch (Sozzani et al., 1998; Palm and Medzhitov,2009), and produce numerous cytokines and chemokines (Takeda K et al., 2003).

Is actually known that miRNAs play an important role in the regulation of innate immunity (Baltimore et al., 2008; O'Connell et al., 2010). Previous studies have demonstrated that miRNAs regulate the inflammatory capacity of the dendritic cells in multiple conditions such as differentiation, maturation and function of the dendritic cells (Smyth et al., 2015).

Recent studies have demonstrated that some natural substance as quercetin can reduce the DCs' activation in response to LPS (Cavalcanti et al., 2014). Quercetin, one of the most common flavonoids present in vegetable and fruits, has a lot of pharmacological properties such as a strong anti-inflammatory activity.

This function has been studied in different cell types, in both animal and human models (Kempuraj et al., 2005; Manjeet and Ghosh, 1999; De Santis et al., 2017).

Recent studies has clearly demonstrated quercetin immunosuppressive effects on dendritic cells function (Huang et al., 2010; Cavalcanti et al., 2014; Delvecchio et al., 2015).

Even though, has not yet been clarified miRNAs role in the cascade of events that leads to the immunosuppressive effect of quercetin in DCs.

The present document illustrates, for the first time, the miRNA expression profile of LPS-stimulated bone marrow dendritic cells, after quercetin exposure, using next-generation sequencing (NGS). A specific group of 113 miRNAs that were differentially regulated in LPS-stimulated DCs, after quercetin exposure, along with their candidate target genes it has been identified. Among these miRNAs, miR-369-3p highlighted a strong expression after quercetin exposure; furthermore its putative target genes were C/EBP-β and TNFα, two pivotal genes for the inflammatory response by LPS.
C/EBP-β gene belongs to a family of transcription factors involved in the regulation and expression of proinflammatory cytokines during macrophage activation and the acute phase response (Takiguchi, 1998; Niehof et al., 2001). Inflammatory stimuli phosphorylate the transactivation domain of C/EBP-β and increase the transcription of the C/EBP-β gene (Trautwein et al., 1993). In turn, C/EBP-β promotes the expression of various proinflammatory genes such as IL-1, IL-6, IL-8, TNF-β (Poli, 1998; Natsuka et al., 1992; Scott et al., 1992).

The inhibitory effect of quercetin on C/EBP-β expression was recently described in intestinal epithelial cells (Hungness et al., 2002; Shimizu et al. 2015) but its effects in DCs are unknown.

Several patent documents referring to therapeutic properties relates to the use of miRNA, are known in the state of the art.

The patent document WO2018008774 relates to a pharmaceutical composition for the prevention and treatment of metabolic diseases based on the miR-7, miR-18a or miR-18b, as an active ingredient; this document does not refer to the use of miR-369-3p neither to its use as anti-inflammatory agent.

The patent KR20170017519 relates to pharmaceutical composition for treating inflammatory bone diseases based on miR-218-2; said miRNA is involved in the expression of inflammatory mediators and inhibits expression of receptors which induce osteoclasts differentiation. This document does not show the role of miR-369-3p in the regulation of innate immunity mediated by dendritic cells, neither the miR-369-3p relationship between the suppression of chronic inflammatory response by means of regulation of C/EBP-β gene and the reduction of TNFα and IL-6.

The patent document US2013225440 describes to a series of miRNA associated with the treatment of the inflammatory bowel diseases; this document does not illustrate the role of miR-369-3p associated with dendritic cells activity and, then, with innate immunity. This document does not show, in any way, the relation between miRNAs, and more specifically miR-369-3p, and inflamed tissues; in inflamed tissues the miR-369-3p resulted decreased in comparison with non-inflamed tissue (the relationship between miR-369-3p and the inflammatory cascade in bowel disease are demonstrated in the analysis of biopsies).

Furthermore, in the technical field of reference, there is the need to have a pharmaceutical composition which, in the light of the relationship between miR-369-3p and dendritic cells deputed to innate immunity and, in the light of the relationship between miR-369-3p and its increasing in concentration in healthy tissues, use miR-369-3p as an active ingredient for the treatment of chronic inflammatory diseases.

The aim of the present invention is to provide a pharmaceutical composition that specifically modulates the mediators of inflammation in chronic inflammatory diseases, and more specifically in chronic inflammatory bowel diseases so as to also reduce side effects and overcome the currently widespread approach to the treatment of diseases chronic intestinal inflammatory which aims mainly to control symptoms and increase quality of life.

In particular, aim of the present invention is to describe the unexpected role of miR-369-3p in the regulation of the inflammatory response affecting the production of inflammatory cytokines, in particular, TNFα and IL-6.

Another aim of the present invention is to provide the administration of this pharmaceutical composition to patients with chronic inflammatory bowel diseases that do not respond to the other pharmaceutical compositions administration based on biological active ingredients currently used.

The present invention provides, then, an active ingredient for treating inflammatory diseases, which is miR-369-3p.

Further features of the present invention, including the embodiments, the medical use and the anti-inflammatory effects are described in the following attached claims.

The aforementioned claims are reproduced below.

### Brief description of the invention

Below is provided a description of a pharmaceutical composition based on miR-369-3p as active ingredient which shows an unexpected anti-inflammatory activity regulation mediated by dendritic cells, in particular bone marrow derived dendritic cells, more in particular murine bone marrow derived dendritic cells. This pharmaceutical composition comprising miR-369-3p as active ingredient shows ability in regulating the expression of the gene C/EBP-β inducing a reduction in the production of inflammatory cytokines as TNFα e IL-6.

The mechanism of action of said pharmaceutical composition comprising miR-369-3p as active ingredient provides that the level of miR-369-3p decreases in the inflamed areas and increase in non-inflamed, healthy, areas. In particular, the level of miR-369-3p in human biopsy samples taken from inflamed areas of patients with inflammatory bowel disease (IBD - Inflammatory Bowel Disease) is decreased, and vice versa, increased in healthy areas.

Said active ingredient of the pharmaceutical composition according to present invention, miR-369-3p, in vitro cellular transfection experiments on dendritic cells, more in particular in murine bone marrow derived dendritic cells, shows a reduction in C/EBP-β expression after LPS stimulation.

Therefore, the present invention provides a pharmaceutical composition based on one or more miRNAs and having as active ingredient miR-369-3p, for treating the chronic inflammatory diseases, in particular the inflammatory bowel disease, more in particular ulcerative colitis, Chron's disease, Colitis-Associated Colorectal Cancer, rheumatoid arthritis, and finally dermatitis and psoriasis.

The present invention will be more evident in the follow detailed description of the invention and in experiments with reference to enclosed drawings.

### Description of figures

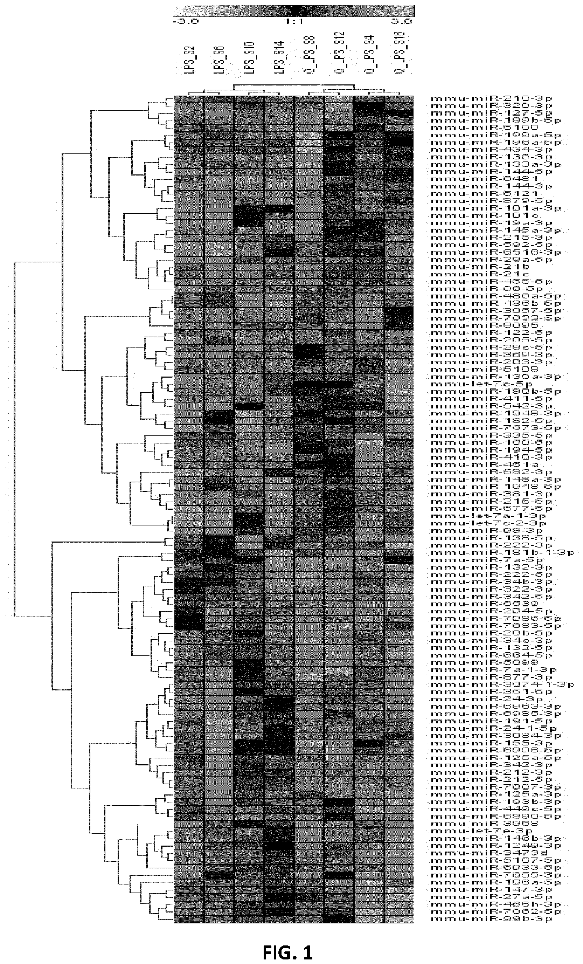

Figure 1 shows a bi-dimensional clustering based on data expression profiles using the 113 significantly differentially expressed miRNAs (FDR ≤ 0.05 and fold change threshold > 2) in two examined groups: LPS-stimulated DCs, treated and not-treated with quercetin. Each column represents a sample and each row a miRNA.
   The expression level of the 113 miRNAs identified is represented in the top miRNA group, from left to right as less to more expressed, and in the lower miRNA group, from more to less expressed.
   The respective miRNA symbols are shown on the right side. Clustering analysis based on the differential expression of miRNAs highlighted the clear distinction between two main miRNA groups that perfectly differentiate the two analyzed groups.

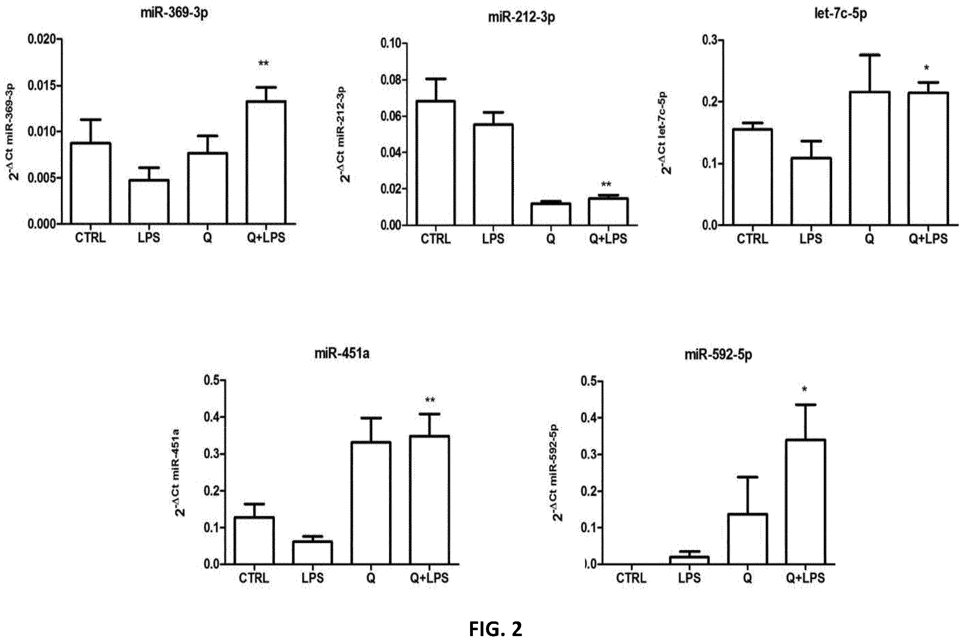

Figure 2 shows the results of a real-time PCR analysis (qRT-PCR) in order to validate the results obtained from the differentially expressed miRNAs sequencing assays. Expression levels of the following miRNAs that are miR-369-3p, miR-212-3p, let-7c-5p, miR-451a and miR-592-5p have been analyzed. miRNAs expression levels were quantified using qRT-PCR and the relative expression was normalized to the geometric mean of two endogenous controls (miR-186-5p and miR-26a-5p). All analyzed miRNAs showed a significant change in expression in LPS-stimulated murine bone-marrow dendritic cells (BMDCs) after quercetin treatment, confirming the sequencing results. *p< 0.05; **p<0.01 compared to treatment with LPS.

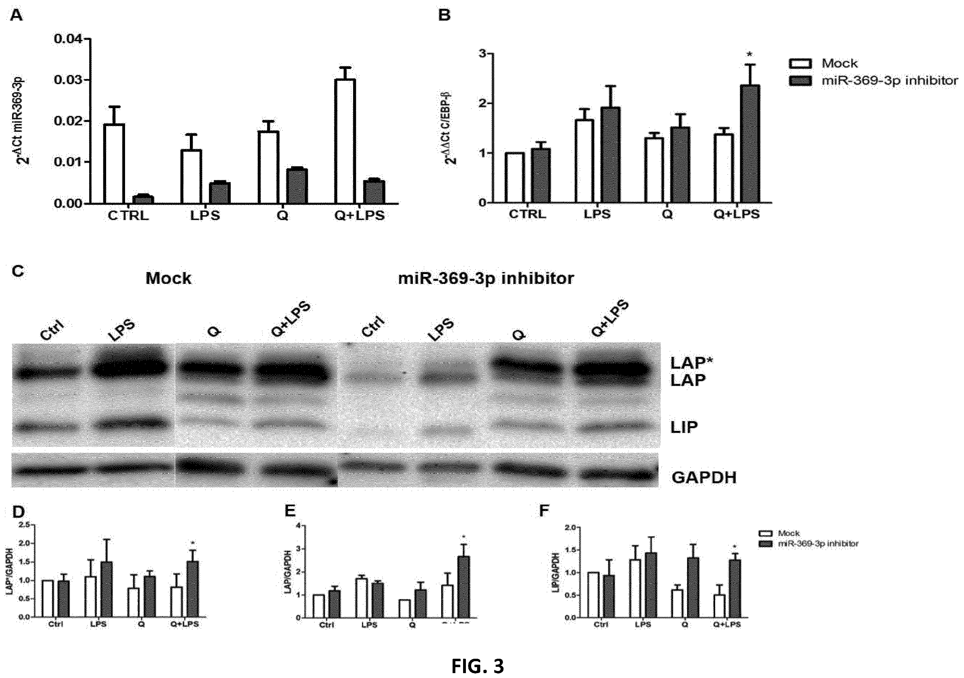

Figure 3 shows the effect of in vitro transfection of inhibitor molecules of miR-369-3p in murine bone-marrow dendritic cells in all conditions. (A) Illustrates a histogram showing miR-369-3p levels determined by qRT-PCR and normalized to the geometric mean of two endogenous controls (miR-186-5p and miR-26a-5p). (B) Illustrates a histogram showing C/EBP-β expression levels analyzed by qRT-PCR in quercetin-treated and not treated DCs, stimulated with 1 µg/mL of LPS for 6 hours after transfection with 75 nM of miR-369-3p inhibitor. Silencing the activity of miR-369-3p within LPS-stimulated DCs after quercetin treatment, the endogenous amount of C/EBP-β was increased 1.7-fold. (C) Illustrates the results obtained by means of Western blot assay representing protein levels of C/EBP-β in quercetin-treated and not treated DCs stimulated with 1 µg/mL of LPS for 24 hours after transfection with 75 nM of miR-369-3p inhibitor. (D-E-F) Illustrate a histogram showing a significant increase in C/EBP-β protein production (in all isoforms) after transfection.

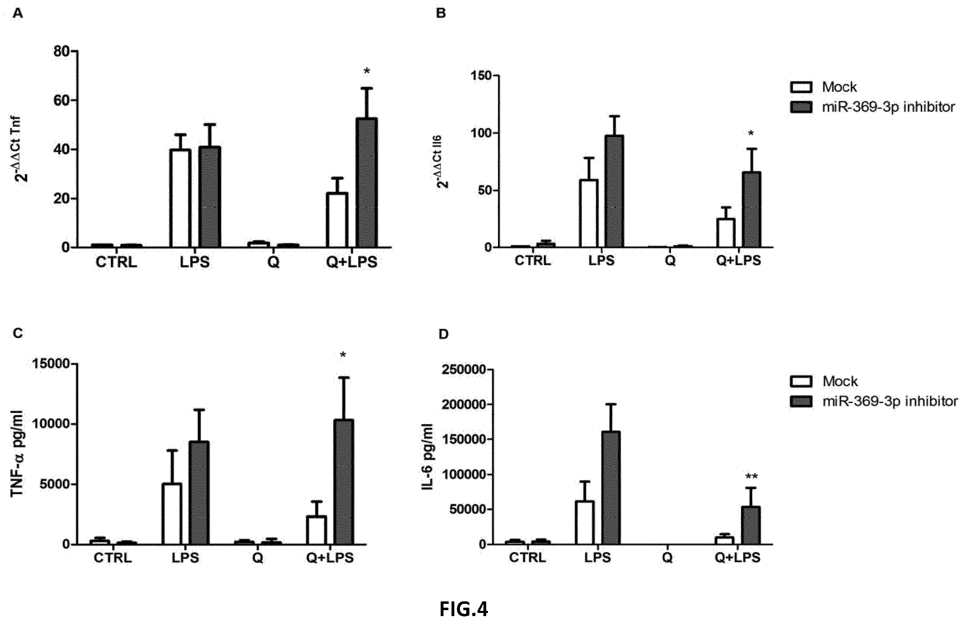

Figure 4 shows histograms illustrating the effects of miR-369-3p inhibitor transfection on TNFα and IL6 production. miR-369-3p inhibition in LPS-stimulated DCs treated and not with quercetin led to a significant increase of TNFα and IL6 at genetic level (Figures A-B) and protein level (Figures C-D).

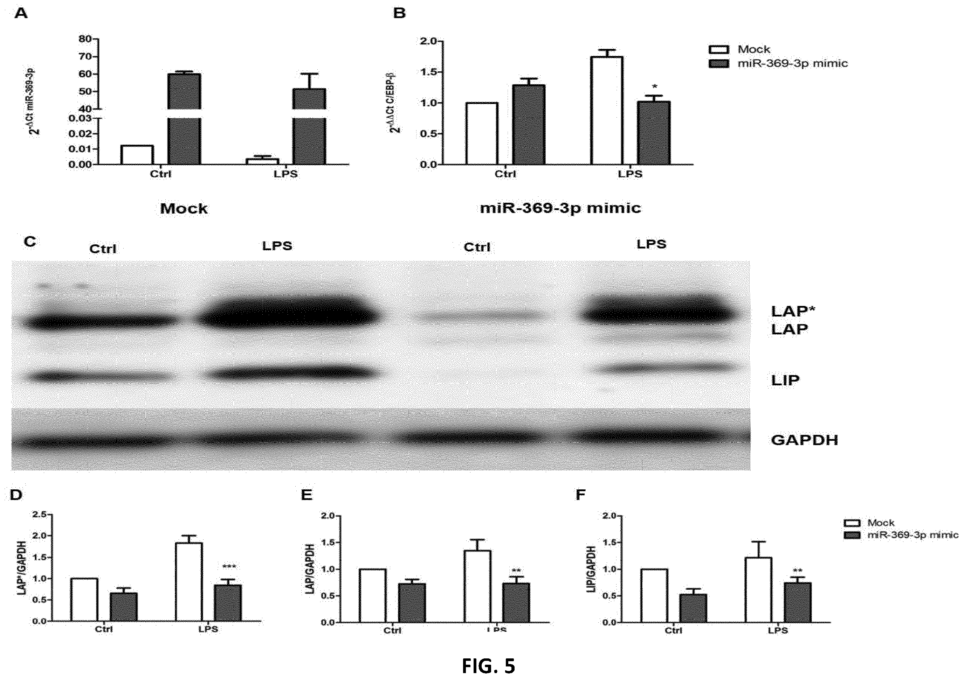

Figure 5 shows the transfection effect of molecules of miR-369-3p in all the conditions. (A) Illustrates a histogram showing miR-369-3p levels determined by qRT-PCR and normalized to the geometric mean of two endogenous controls (miR-186-5p and miR-26a-5p). (B) Illustrates a histogram showing C/EBP-β expression levels analyzed by qRT-PCR in DCs stimulated with 1 µg/mL of LPS for 6 hour after transfection with 50 nM of miR-369-3p mimic. When the endogenous quantity of miR-369-3p within LPS-stimulated DCs was increased, the endogenous amount of C/EBP-β was decreased 1.7-fold. (C) Illustrates Western blot assay results showing protein levels of C/EBP-β in DCs stimulated with 1 µg/mL of LPS for 24h after transfection with 50 nM of miR-369-3p mimic. (D-E-F) Illustrate histograms relating to significant decreasing in C/EBP-β protein production (in all isoforms) after transfection.

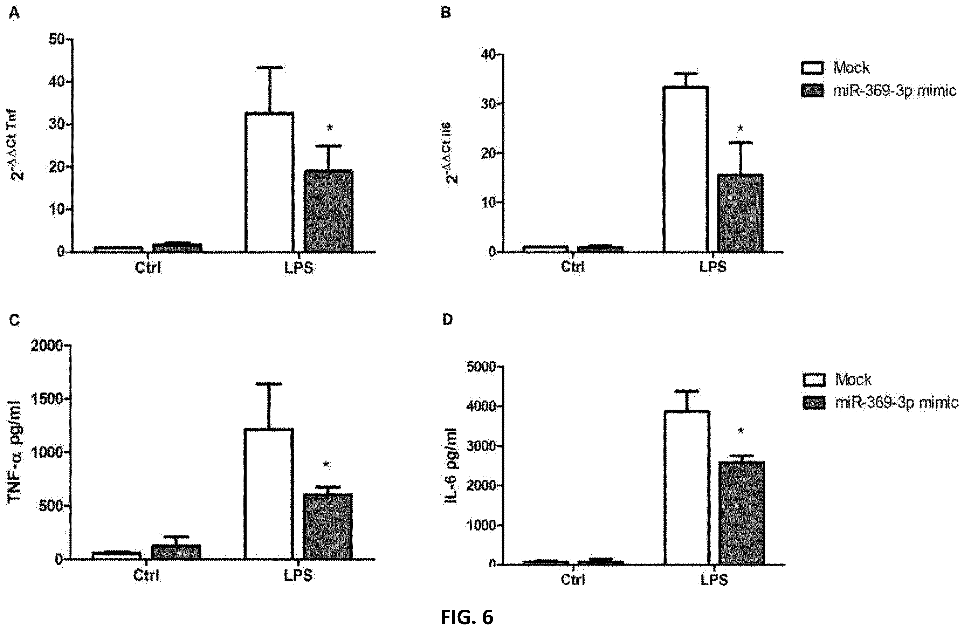

Figure 6 illustrates histograms showing the effect of miR-369-3p upregulation in LPS-stimulated dendritic cells after transfection with miR-369-3p mimic; figures A and B show a significant decrease of TNFα and IL6 expression at mRNA level; figures C and D shows a significant decrease of TNFα and IL6 expression at protein level (C-D).

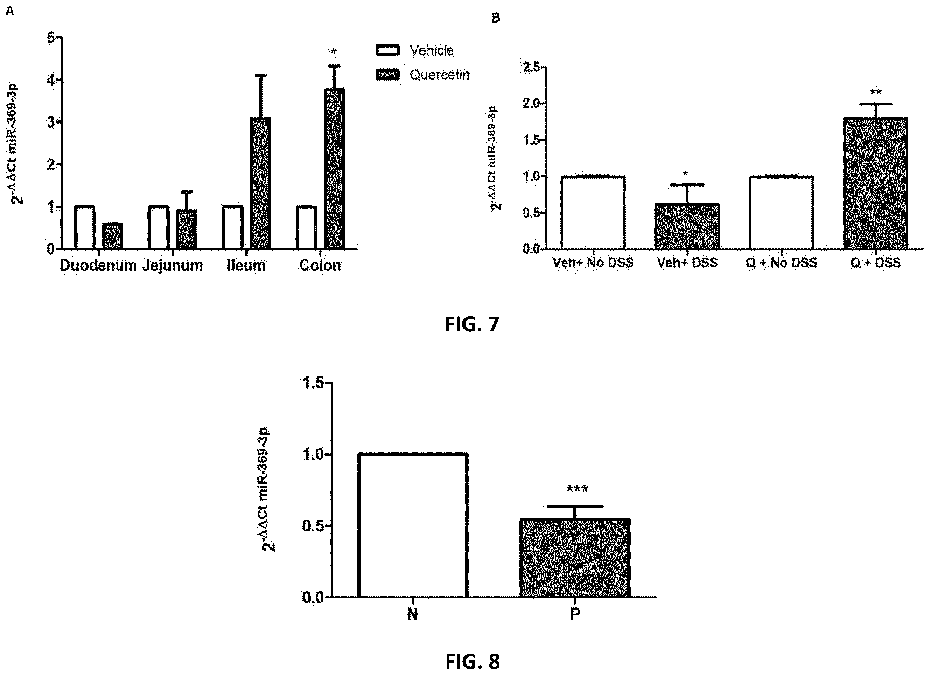

Figure 7 illustrates histograms showing the effect of quercetin or vehicle administration in mice with DSS-induced colitis. (A) Illustrates miR-369-3p expression levels measured by means of qRT-PCR in the duodenum, jejunum, ileum and colon of mice treated with oral administrated quercetin. miR-369-3p expression levels were significantly increased only in the colon (3.7-fold increase; * p< 0.05). (B) Illustrates miR-369-3p expression levels measured by means of qRT-PCR in colon in which acute colitis was induced after administration of 2% DSS in drinking water with administration of quercetin or vehicle. miR-369-3p expression levels were significantly decreased in DSS condition without quercetin treatment (*p<0.05). Oral administration of quercetin was significantly increased miR-369-3p expression levels (**p<0.01).
Figure 8 illustrates histograms showing the results of miR-369-3p expression levels in total RNA samples isolated from non-inflamed and inflamed regions from biopsy samples of IBD patients. miR-369-3p expression levels in the inflamed areas was significantly lower than those in non-inflamed tissue (1.85-fold decrease, p <0.001). For each patient, the relative miR-369-3p levels of inflamed regions were compared to non-inflamed areas. Expression data were normalized to the geometric mean of two endogenous controls (miR-186-5p and miR-26a-5p). The histograms represent the mean ± SEM. *** p< 0.001.

### Detailed description of the invention

The present invention describes a pharmaceutical composition based on one or more miRNAs, at least one pharmaceutically acceptable vehicle and/or diluent and/or excipient, characterized by the fact that comprises miR-369-3p as active ingredient. Said active ingredient, miR-369-3p, has been selected from a group of miRNA (113 miRNA) differentially expressed in LPS stimulated bone-marrow derived dendritic cells with and without quercetin exposure starting from a sequencing assay. Some of these 113 miRNAs show a different up-regulation or down-regulation after treatment with quercetin, in other term quercetin modulates their miRNAs expression; among these, as shown in Table 1 here below, the miR-369-3p shows significant up-regulation activity such as a significant expression after quercetin exposure; moreover, miR-369-3p was selected in a miRNA group regulating genes involved in the inflammation, as shown in bioinformatics analysis.

**Table 1. List of 113 miRNAs differently expressed in LPS treated DCs exposed or not to quercetin using generation sequencing technology with fold-change and False Discovery Rate (FDR).**

| Nome miRNA | Fold-change | FDR |
|---|---|---|
| mmu-miR-215-5p | 9.405 | 3.84E-24 |
| mmu-miR-144-3p | 9.188 | 1.65E-12 |
| mmu-miR-451a | 8.152 | 9.83E-14 |
| mmu-miR-5108 | 6.224 | 9.63E-03 |
| mmu-miR-144-5p | 5.904 | 3.49E-04 |
| mmu-miR-145a-3p | 5.851 | 2.93E-04 |
| mmu-miR-199b-5p | 5.499 | 1.72E-02 |
| mmu-miR-127-5p | 5.421 | 4.39E-03 |
| mmu-miR-5121 | 5.085 | 5.67E-16 |
| mmu-miR-7033-5p | 4.972 | 4.94E-09 |
| mmu-miR-196a-5p | 4.892 | 1.67E-02 |
| mmu-miR-8095 | 4.482 | 6.23E-03 |
| mmu-miR-136-3p | 4.433 | 8.20E-03 |
| mmu-miR-203-3p | 4.161 | 1.35E-06 |
| mmu-miR-6481 | 4.152 | 5.12E-04 |
| mmu-miR-7673-5p | 4.122 | 4.84E-02 |
| mmu-miR-133a-3p | 4.115 | 4.39E-03 |
| mmu-miR-369-3p | 3.999 | 1.09E-05 |
| mmu-miR-434-3p | 3.994 | 2.54E-02 |
| mmu-miR-5100 | 3.855 | 4.22E-02 |
| mmu-miR-210-3p | 3.821 | 2.52E-02 |
| mmu-miR-381-3p | 3.782 | 7.78E-03 |
| mmu-miR-215-3p | 3.773 | 2.52E-02 |
| mmu-miR-410-3p | 3.746 | 2.31E-04 |
| mmu-miR-199a-5p | 3.684 | 1.20E-02 |
| mmu-miR-411-5p | 3.256 | 9.86E-05 |
| mmu-miR-21c | 3.124 | 2.35E-03 |
| mmu-miR-335-5p | 3.115 | 1.18E-02 |
| mmu-miR-29a-5p | 3.075 | 1.45E-03 |
| mmu-miR-21b | 3.027 | 1.45E-03 |
| mmu-miR-879-5p | 3.007 | 5.97E-04 |
| mmu-miR-677-5p | 2.978 | 3.92E-05 |
| mmu-miR-592-5p | 2.880 | 9.16E-03 |
| mmu-miR-6516-3p | 2.815 | 4.74E-02 |
| mmu-miR-19a-3p | 2.782 | 1.18E-02 |
| mmu-miR-205-5p | 2.764 | 4.22E-02 |
| mmu-miR-486a-5p | 2.722 | 1.36E-02 |
| mmu-miR-486b-5p | 2.722 | 1.36E-02 |
| mmu-miR-100-5p | 2.680 | 1.78E-02 |
| mmu-miR-122-5p | 2.602 | 1.74E-02 |
| mmu-miR-3057-5p | 2.545 | 2.05E-06 |
| mmu-miR-190b-5p | 2.534 | 9.51E-03 |
| mmu-miR-96-5p | 2.511 | 1.51E-02 |
| mmu-miR-101a-3p | 2.451 | 3.50E-02 |
| mmu-miR-101c | 2.451 | 3.50E-02 |
| mmu-miR-455-5p | 2.223 | 1.35E-03 |
| mmu-miR-182-5p | 2.147 | 1.83E-02 |
| mmu-let-7c-5p | 2.146 | 4.39E-03 |
| mmu-miR-1948-5p | 2.099 | 3.02E-02 |
| mmu-miR-542-3p | 1.987 | 2.72E-02 |
| mmu-miR-98-3p | 1.889 | 6.88E-03 |
| mmu-miR-29c-5p | 1.868 | 4.24E-03 |
| mmu-miR-320-3p | 1.862 | 4.74E-02 |
| mmu-miR-130a-3p | 1.839 | 2.52E-02 |
| mmu-miR-148a-3p | 1.764 | 1.76E-02 |
| mmu-miR-1948-3p | 1.700 | 3.58E-02 |
| mmu-let-7a-1-3p | 1.698 | 3.50E-02 |
| mmu-let-7c-2-3p | 1.698 | 3.50E-02 |
| mmu-miR-194-5p | 1.693 | 9.16E-03 |
| mmu-miR-582-3p | 1.615 | 3.99E-02 |
| mmu-miR-34b-3p | -1.715 | 3.50E-02 |
| mmu-miR-7683-5p | -1.765 | 2.54E-02 |
| mmu-miR-322-3p | -1.782 | 4.39E-03 |
| mmu-miR-7a-1-3p | -1.862 | 2.12E-02 |
| mmu-miR-7a-5p | -2.019 | 4.02E-02 |
| mmu-miR-664-5p | -2.034 | 4.82E-05 |
| mmu-miR-155-3p | -2.054 | 4.22E-02 |
| mmu-miR-222-5p | -2.108 | 2.93E-04 |
| mmu-miR-222-3p | -2.138 | 2.03E-02 |
| mmu-miR-24-3p | -2.139 | 2.54E-02 |
| mmu-miR-20b-5p | -2.222 | 9.03E-03 |
| mmu-miR-24-1-5p | -2.241 | 2.68E-02 |
| mmu-miR-181b-1-3p | -2.260 | 1.51E-02 |
| mmu-miR-106a-5p | -2.305 | 4.24E-03 |
| mmu-miR-3084-3p | -2.313 | 4.74E-02 |
| mmu-miR-27a-5p | -2.325 | 3.31E-02 |
| mmu-miR-193b-3p | -2.402 | 4.39E-02 |
| mmu-miR-125a-5p | -2.508 | 9.75E-04 |
| mmu-miR-877-3p | -2.515 | 2.08E-03 |
| mmu-miR-7062-5p | -2.546 | 2.98E-02 |
| mmu-miR-146b-3p | -2.561 | 2.31E-04 |
| mmu-miR-34c-3p | -2.605 | 2.16E-04 |
| mmu-miR-191-5p | -2.651 | 1.83E-02 |
| mmu-miR-99b-3p | -2.706 | 3.54E-02 |
| mmu-miR-5099 | -2.758 | 2.36E-07 |
| mmu-miR-3074-1-3p | -2.760 | 2.26E-02 |
| mmu-miR-342-5p | -2.792 | 1.88E-07 |
| mmu-miR-7655-3p | -2.830 | 3.60E-02 |
| mmu-miR-147-3p | -2.865 | 5.85E-04 |
| mmu-miR-466h-3p | -2.924 | 9.39E-03 |
| mmu-miR-351-5p | -3.040 | 5.90E-03 |
| mmu-miR-342-3p | -3.103 | 8.86E-08 |
| mmu-miR-6963-3p | -3.205 | 7.72E-03 |
| mmu-miR-449c-5p | -3.334 | 2.26E-02 |
| mmu-miR-3473d | -3.488 | 9.36E-04 |
| mmu-miR-6933-5p | -3.503 | 1.76E-02 |
| mmu-miR-1249-3p | -3.503 | 4.24E-03 |
| mmu-miR-132-3p | -3.549 | 2.26E-11 |
| mmu-miR-138-5p | -3.773 | 4.24E-03 |
| mmu-miR-132-5p | -3.839 | 7.09E-09 |
| mmu-miR-6985-3p | -3.881 | 3.53E-02 |
| mmu-miR-6990-5p | -3.907 | 4.92E-02 |
| mmu-miR-125a-3p | -3.970 | 1.15E-02 |
| mmu-miR-204-5p | -4.028 | 5.31E-05 |
| mmu-let-7e-3p | -4.364 | 5.80E-03 |
| mmu-miR-7086-5p | -4.502 | 4.45E-03 |
| mmu-miR-6996-5p | -4.720 | 6.23E-03 |
| mmu-miR-3968 | -5.006 | 3.27E-06 |
| mmu-miR-212-5p | -6.180 | 1.42E-08 |
| mmu-miR-5107-5p | -6.733 | 7.87E-05 |
| mmu-miR-6539 | -7.124 | 8.86E-08 |
| mmu-miR-7007-3p | -8.421 | 9.88E-07 |
| mmu-miR-212-3p | -8.520 | 1.51E-20 |

The pharmaceutical composition, according to the invention, may include one or more miRNAs. Said miRNAs may belong to the group comprising the 113 tested miRNAs. The pharmaceutical composition, according to the invention, may include one or more pharmaceutically acceptable additive as preservatives, antioxidants, acidifiers, chelator agents, perfumes, rheological additives, solubilizers or neutralizing agents.

In particular, the aforementioned pharmaceutically acceptable additives belong to the group which comprises: imidazolidinyl urea, benzoic acid or its salts or esters, sorbic acid or its salts, 4-hydroxybenzoic acid or its salts or esters, phenoxyethanol, dimethylol dimethylhydantoin (DMDM hydantoin), benzyl alcohol, isothiazolinones, dehydroacetic acid and sodium salt, parabens, vitamins (group A, C, E), lecithins, arginine, soda, citric acid, lactic acid, carbomer and their derivatives, hydroxyethylcellulose, xanthan gum, polymers acrylics or EDTA can be used in an advantageous way.

The pharmaceutical composition, according to the invention, may contain one or more moisturizers, emollients, surfactants and emulsifying agents; the aforementioned pharmaceutically acceptable vehicles are selected from the group which comprises: glycerin, butylene glycol, pentylene glycol, hydroxyphenylpropylamobenzoic acid, 4-tert-butylcyclohexanol, polyoxyethylene stearate, glyceryl stearate, dimethicone, phenyl trimeticone, panthenol, synthetic or natural triglycerides, esters of fatty acids, glycol stearate or their esters, alcohol cetylstearyl, plant extracts, bisabolol, aloe vera, vegetable oils, elastin or collagen.

The solvent used to bring the final weight of the composition up to 100% is selected from among water, alcohols, glycols, oils selected from those normally used in the field of cosmetics and pharmaceuticals.

The pharmaceutical composition can be in the form of a liquid or powder, dried and freeze-dried.

The pharmaceutical composition is administered as a medicament in solid form such as tablets, pills, hard capsules, powders, granules, suppository drugs, in semi-solid form such as gels, ointments, lubricants, pastes, in liquid form such as drinks, syrups, vials, drops, eye drops.

The pharmaceutical composition, according to the invention, is characterized by comprising the miR-369-3p as active ingredient.

The pharmaceutical composition, according to the invention, based on miR-369-3p is capable to reduce the activity of C/EBP-β gene with a consequent reduction of TNFα and IL-6 production.

The study of this invention started from sequencing analysis followed by bioinformatic analysis, performed by inventors, in which it has been demonstrated that in LPS-stimulated murine bone-marrow derived dendritic cells, mRNA levels in relation to gene expression of C/EBP-β, TNFα and IL-6 levels are regulated by quercetin exposure. It has also demonstrated that the functional inhibition of miR-369-3p in dendritic cells after quercetin exposure, led to a reduction of C/EBP-β gene at mRNA and protein level biologically confirming the bioinformatic data. Therefore, the loss of activity of miR-369-3p in BMDC cells stimulated with LPS after quercetin exposure led to an increase of TNFα and IL-6 production.

The studies of inventors have demonstrated that LPS-treated DCs previously exposed to quercetin, overexpress miR-369-p. This modulation, in turn, downregulate C/EBP-β, thus resulting in a lower production of inflammatory cytokines, in particular TNFα and IL-6.

To further confirm the protective role of miR-369-3p in LPS-stimulated DCs, similarly to the results of quercetin exposure, it has been noted that the ectopic induction of miR-369-3p suppresses the inflammatory response after LPS exposure, acting on C/EBP-β expression and, then, on TNFα and IL-6 production.

The inventors have also demonstrated, regarding the intestine, that in vivo oral administration of quercetin resulted in an increase of miR-369-3p expression levels in the colon of treated mice. The same increase it has also been observed in DSS treated mice after oral administration of quercetin. This result suggests that the observed quercetin-mediated protection against chronic inflammatory syndromes could be the result of miR-369-3p upregulation.

Therefore the role of miR-369-3p in chronic inflammatory syndromes may be the result of its down-regulation; the involvement of miR-369-3p in the regulation of the inflammatory cascade in chronic inflammatory bowel disease has also been demonstrated in human biopsies in which, following the comparison of miR-369-3p levels in inflamed areas with respect to the non-inflamed areas, it has been shown that the level of miR-369-3p were decreased in human inflamed regions of intestine compared to non-inflamed regions.

To determine the expression levels of miR-369-3p in tissue from IBD patients, it has been analyzed RNA samples from non-inflamed and inflamed regions of intestine obtained from these patients. As shown in Figure 8, following the comparison of miR-369-3p levels in inflamed areas with respect to the non-inflamed areas, it can be shown that miR-369-3p in the inflamed areas was significantly lower than those in non-inflamed tissue (1.85-fold decrease, p <0.001). This result confirms the involvement of miR-369-3p in the regulation of the inflammatory cascade, in particular in inflammatory bowel disease.

The pharmaceutical composition, according to the invention, is indicated as a medicament for using in the treatment of chronic inflammatory disease; in particular for the treatment of the inflammatory bowel disease and more in particular ulcerative colitis, Chron's disease, Colitis-Associated Colorectal Cancer, rheumatoid arthritis, dermatitis and psoriasis.

In particular, said pharmaceutical composition has activity on certain immune innate cells as dendritic cells as demonstrated in the following experiments performed on murine bone-marrow derived dendritic cells; said composition has a particular activity on tissues characterized by a strong dendritic cells composition as intestine tissue and skin.

The pharmaceutical composition, according to the invention, may be administered with at least one active ingredient known used for treating chronic inflammatory diseases. The pharmaceutical composition, according to the invention, may be produced in such a way as to allow release of the various active ingredients in a simultaneous, sequential or delayed manner.

The present invention provides a pharmaceutical composition that can be administered both to human and animal models.

The present invention provides also an active ingredient, that is the miR-369-3p for using in the treatment of inflammatory diseases based on a disfunction of immune innate cells as dendritic cells, in particular for using in the treatment of chronic inflammatory diseases.

Finally, the present invention provides a method to determine the expression of miR-369-3p in the inflammatory bowel disease comprising the following steps:
- withdrawing biopsy specimens from inflamed and non-inflamed intestinal regions;
- measuring miR-369-3p expression levels in inflamed regions;
- measuring miR-369-3p expression levels in non-inflamed regions;
- comparing miR-369-3p expression levels between inflamed and non-inflamed regions;
- verifying of decreased miR-369-3p levels in inflamed regions.

The method for determining the activity of miR-369-3p in inflammatory bowel disease, by comparing the levels of miR-369-3p measured in the inflamed areas with those of the non-inflamed areas, allows to verify that the decrease in miR-369-3p levels in inflamed areas is indicative of miR-369-3p activity in determining inflammatory bowel disease. The method allows the correlation between the decrease of the miR-369-3p level in the intestinal tissues with chronic inflammatory bowel disease and provides a predictive evaluation of inflammatory bowel disease based on the decreased levels of miR-369-3p in intestinal tissue.

The invention is exemplified by the following and non-limiting experiments.

### Experiment 1

The role of differentially expressed miRNAs in LPS-treated bone-marrow derived dendritic cells exposed or not to quercetin has been investigated in the first experiment. Bone marrow (BM) derived DCs (BMDCs) were obtained from C57BL/6 mice. Briefly, BM cells from the tibiae and femurs of 6- to 8-week-old male mice were flushed with PBS with 0.5mM EDTA (Thermo Fisher Scientific, MA, USA), and of red blood cells red blood cells have been lysed with ACK lysing buffer (Ammonium-Chloride-Potassium) (Thermo Fisher Scientific, MA, USA). Cells were plated in a 10 ml dish (1x106 cells/mL) in RPMI-1640 (Thermo Fisher Scientific, MA, USA) supplemented with fetal bovine serum heat-inactivated in order to destroy the complement molecules (FBS, Thermo Fisher Scientific, MA, USA), 2mM L-glutamine, 1mM sodium pyruvate, 1 mM nonessential amino acids, 25 mM HEPES, 100 U/mL penicillin (Thermo Fisher Scientific, MA, USA), 100 mg/mL streptomycin (Thermo Fisher Scientific, MA, USA), 25 µg/mL rmGM-CSF (Miltenyi Biotec, Bergisch Gladbach, Germany), and 25 µg/mL rmlL-4 (Miltenyi Biotec, Bergisch Gladbach, Germany) at 37°C in a humidified 5% CO2 atmosphere, harvested, restimulated with new growth factors and plated at 1x10⁶ cells/mL on 12-well culture plates.

Cells were treated with 25uM of natural quercetin (Farmalabor) on day 5 and day 7. On day 8 BMDCs were stimulated with 1 µg/mL of LPS for 6 or 24 hours at 37°C in 5% CO₂ and used respectively for total RNA extraction and protein / supernatant collection.

To identify miRNAs differentially expressed in LPS-treated BMDCs exposed or not to quercetin, it has been analyzed their global expression profile by means NGS. Untreated or quercetin-exposed BMDCs were used as controls.

The number of raw reads obtained per sample ranged from 19,887,893 to 28,765,373 (average = 23,470,539) with a mean quality score of 35.34.

Mature miRNAs identification was performed on all samples using sRNAtoolbox (Rueda et al., 2015). An average of 730 mature miRNAs for each sample was successfully annotated in miRBase 21 (assembly mm9). Differential expression analysis between LPS-treated BMDCs exposed or not to quercetin revealed that there are 113 significantly differentially expressed miRNAs (FDR ≤ 0.05 and fold change threshold > 2).

Clustering analysis generated a dendrogram showing two clearly separated groups (Figure 1).

Subsequently, in the first experiment an in silico bioinformatic analysis of the miRNA target genes was performed. The computational targets of each of these miRNAs were studied using some bioinformatics algorithms such as miRBase 21.1, TargetScan 7.1 (http://www.targetscan.org/vert_71/), miRWalk 2.0 (http://zmf.umm.uniheidelberg.de/apps/zmf/mirwalk2/), miRDB (http://www.mirdb.org/) and TarBase v.8 (http://carolina.imis.athena-innovation.gr/diana_tools/web/index.php?r=tarbasev8/index). To reduce the number of false positive results, five different algorithms were used and we only listed the putative genes predicted by at least two of them.

Based on the results of the bioinformatics analysis it has been identified one of the potential targets of miR-369-3p as the CCAAT/enhancer Binding Protein β (C/EBP-β). Other target genes of miR-369-3p were Tumor Necrosis Factor (TNF), Glycogen Synthase Kinase 3 Beta (GSK3B) and sirtuin 1 genes (SIRT1).

The in-silico analysis showed that the miR-369-3p increase, following quercetin exposure, could result in C/EBP-β downregulation and in a consequent reduction of inflammatory cytokines release. In fact, sequence alignment of murine miR-369-3p (Chr12: 109,741,418-109,745,496) with the 3'-untranslated region (UTR) C/EBP-β identified a binding site (nucleotides 381-388 in murine C/EBP-β) that is well conserved among different species.

### Experiment 2

In the second experiment the miRNAs sequencing results of Figura 1 differentially expressed have been validated by real-time PCR (q-RT-PCR).

Expression levels of the following miRNAs were analyzed: miR-369-3p, miR-212-3p, let-7c-5p, miR-451a and miR-592-5p on miRNAs isolated from BMDCs coming from an independent set of 4 wild-type mice, exposed to vehicle or quercetin and treated with 1 µg/mL of LPS for 6 hours.

The miRNA expression levels were quantified using qRT-PCR and the relative expression was normalized to the geometric mean of two endogenous controls (miR-186-5p and miR-26a-5p).

Total RNA, including small RNA fractions, was reverse transcribed with the TaqMan Advanced miRNA cDNA Synthesis Kit (Life Technologies) following the manufacturer's instructions.

Real-time RT-PCR for the quantification of a group of miRNAs (miR-369-3p, miR-212-3p, let-7d, let-7c-5p, miR-451a and miR-592-5p, plus an endogenous control) was carried out with TaqMan Advanced miRNA assays and with Taq Fast Advanced Master Mix (Life Technologies).

### Experiment 3

The third experiment involved the execution of a series of transfection experiments on mouse bone marrow dendritic cells (BMDC) in order to validate the results of the bioinformatics analysis. The link between quercetin exposure and miR-369-3p activity has never been functionally characterized; therefore, the ability of the miR-369-3p to modulate the expression of the C / EBP-β gene was tested.

Transfection of miRNAs in lymphomonocytes in culture was performed using a cationic liposome-based reagent. Cationic liposomes are artificial lipid vesicles, with a positive electric charge, capable of incorporating negatively charged miRNAs and transporting them into cells by fusion with cell membranes or by receptor-mediated endocytosis. The miRNA, enclosed in the liposome, is subsequently released into the cytoplasm where it goes to increase (mimic) or to reduce (inhibitor) the pre-existing miRNA levels.

The miRNA mimic are synthetic miRNAs pre-designed for insertion of miRNAs pathway at level of mature miRNAs. The miRNA inhibitors molecules are anti-sense miRNAs having the function of "inhibitors" which silence the endogenous cellular miRNA. These anti-sense miRNAs are complementary to the miRNA sequence and, once they have entered the cell, they determine an effective functional alteration of the complementary miRNA.

In the third experiment, the transient transfection experiments have been performed in bone marrow-derived dendritic cells from an independent group of four wild-type mice, treated for 4 days with quercetin and next stimulated with LPS. BMDCs have been transfected with miR-369-3p "inhibitor" molecules and then C/EBP-β, TNFα and IL6 expression, at mRNA and protein level, has been evaluated.

On day 5, dendritic cells were harvested, re-stimulated with new growth factors and plated at a concentration of 1x10⁶ cells/mL on 12-well culture plates in RPMI-1640 (Thermo Fisher Scientific, MA, USA), supplemented with 10% heat-inactivated fetal bovine serum (FBS, Thermo Fisher Scientific, MA, USA), 2mM L-glutamine, 1mM sodium pyruvate, 1 mM nonessential amino acids, 25 mM HEPES. For inhibitor transfection, BMDCs were treated with 12.5 µM of quercetin for four consecutive days (from day 5 to day 8). On day 6, BMDC cells were transfected with 75nM of miR-369-3p inhibitor (Qiagen).

The transfection was carried out using the TransiT-TKO Transfection Reagent (Mirus) in accordance with the manufacturer's procedure. On day 6, for mimic transfection with miR-369-3p mimic, BMDC cells were transfected with 50 nM of miR-369-3p mimic (Qiagen) using the TransiT-TKO Transfection Reagent (Mirus) in accordance with the manufacturer's procedure.

On day 8, BMDC cells were stimulated with 1 µg/mL of LPS for 6 hours or 24 hours at 37°C in 5% CO2 and used for total RNA extraction and protein/supernatant collection, respectively.

In all transfection experiments, a transfection control was performed by subjecting cells to the transfection procedure without adding miRNA (mock control).

After transfection, miR-369-3p levels were determined with qRT-PCR and normalized to the geometric mean of two endogenous controls (miR-186-5p and miR-26a-5p) (Figure 3-A). C/EBP-β expression levels were analyzed by qRT-PCR in quercetin-treated and not treated BMDCs, stimulated with 1 µg/mL of LPS for 6h after transfection with 75 nM of miR-369-3p inhibitor (Figure 3B).

Real-time PCR amplification reactions were performed in 20 µl of final volume on a CFX96 System (Biorad Laboratories, CA, USA). The normalization was performed on the geometric mean of two endogenous controls: miR-186-5p and miR-26a-5p.

For C/EBP-β, TNFα and IL6 expression analysis, total RNA was reverse-transcribed with I Script Reverse Transcription Supermix (BioRad Laboratories, CA, USA) following the manufacturer's instructions. qRT-PCR amplification reactions were performed in triplicate in 25µl final volumes using SYBR Green chemistry on a CFX96 System (Biorad Laboratories, CA, USA). qRT-PCR was performed using the QuantiTect Primer Assay (Qiagen) and SsoAdvanced Universal SYBR Green Supermix (BioRad Laboratories, CA, USA).

Genes were amplified according to the manufacturer's directions. The Gapdh gene amplification was used as reference standard to normalize the target signal. Comparative real-time PCR for miRNAs and mRNAs was performed in triplicate, including no-template controls.

Relative expression was calculated using the 2-ΔCt method.

Silencing the activity of miR-369-3p within LPS-stimulated BMDCs after quercetin treatment, the endogenous amount of C/EBP-β was increased 1.7-fold. Western blot assay represent protein levels of C/EBP-β in quercetin-treated and not treated BMDCs stimulated with 1 µg/mL of LPS for 24h after transfection with 75 nM of miR-369-3p inhibitor (Figure 3C).

A significant increase in C/EBP-β protein production (in all isoforms) was shown after transfection (Figure 3 D-E-F).

The transfection of BMDCs with 75 nM of miR-369-3p inhibitor led to a statistically significant increase of endogenous mRNA C/EBP-β levels compared to controls (mock) (p =0.03, Figure 3B).

The amount of C/EBP-β after transfection, was determined by Western blotting assay. Total protein extract was prepared with lysis buffer containing 150 mM NaCl, 50 mM Tris-HCI (pH 7.5), 5 mM EDTA, 1.50% NonidetP-40, 0.1% sodium deoxycholate, 1mM sodium orthovanadate, plus cocktail proteinase inhibitors (Sigma Aldrich P8340).

The protein concentration was determined by standard Bradford colorimetric assay (Bio-Rad Laboratories, CA, USA). 60 µg of each protein lysate were separated on Criterion TGX Stain-Free Gels, 4-20% (Bio-Rad Laboratories, CA, USA) and then electrotransferred onto PVDF membrane (Bio-Rad Laboratories, CA, USA). The membranes were incubated in 2% nonfat milk powder diluted in PBS containing 0.1% Tween-20 for 1 hours at room temperature and probed with a monoclonal anti-C/EBP-β antibody (Santa Cruz Biotechnology) in blocking buffer overnight at 4°C. Immunocomplexes were detected with a chemiluminescence method (Bio-Rad Laboratories, CA, USA) using the ChemiDoc instrument (Bio-Rad Laboratories, CA, USA).

The same membranes were stripped and reprobed with anti-GAPDH monoclonal antibody (Santa Cruz Biotechnology). Images were quantified by Image J Software (http://rsb.info.nih.gov/ij). C/EBP-β expression in each sample was normalized divided the intensity of C/EBP-β bands with GAPDH bands.

The ratio was calculated by dividing the normalized values of stimulated and/or transfected cells by the values of control cells.

### Experiment 4

In order to further verify the link between the quercetin-mediated expression of miR-369-3p and quercetin anti-inflammatory properties, it has been tested whether miR-369-3p upregulation could revert the suppression of inflammatory cytokines release. Dendritic cells transfection with 75 nM of miR-369-3p inhibitor caused an increase of TNFα and IL6 genes expression (p < 0.05, Figure 4A-B). Accordingly, following quercetin exposure, DCs transfected with miR-369-3p inhibitor showed an increase in TNFα and IL6 release in the supernatant (p<0.05, Figure 4C-D).

### Experiment 5

To further confirm miR-369-3p as a mediator of quercetin anti-inflammatory activity, it has been performed a transfection reverse experiment in which a short RNA sequence capable to mimic the action of miR-369-3p was used without the use of quercetin. BMDC cells isolated from an independent group of four wild-type mice were transiently transfected with miR-369-3p mimic molecules at the final concentration of 50 nM and subsequently stimulated with LPS.

As shown in Figure 5A, after transfection, miR-369-3p levels were high in all conditions. The experiment show that the expression of the C/EBP-β gene was significantly reduced in BMDC cells stimulated with LPS after transfection with the miR-369-3p mimic compared to the control (mock) (p <0.01, Figure 5 -B).

Moreover, the experiment also allowed to evaluate C/EBP-β protein expression in miR-369-3p mimic transfected BMDC cells from four other wild-type mice. The transient increase in the miR-369-3p level in BMDCs showed a remarkable reduction in C/EBP-β protein expression in all three isoforms as revealed by Western blot (Figure 5 C-D-E-F). The amount of C/EBP-β after transfection, was determined by Western blotting analysis. Total protein extract was prepared with lysis buffer containing 150 mM NaCl, 50 mM Tris-HCI (pH 7.5), 5 mM EDTA, 1.50% NonidetP-40, 0.1% sodium deoxycholate, 1mM sodium orthovanadate, plus cocktail proteinase inhibitors (Sigma Aldrich P8340).

The protein concentration was determined by standard Bradford colorimetric assay (Bio-Rad Laboratories, CA, USA). Sixty µg of each protein lysate were separated on Criterion TGX Stain-Free Gels, 4-20% (Bio-Rad Laboratories, CA, USA) and then electrotransferred onto PVDF membrane (Bio-Rad Laboratories, CA, USA). The membranes were incubated in 2% nonfat milk powder diluted in PBS containing 0.1% Tween-20 for 1 hours at room temperature and probed with a mouse monoclonal anti-C/EBP-β antibody (Santa Cruz Biotechnology) in blocking buffer overnight at 4°C. Immunocomplexes were detected with an enhanced chemiluminescence method (Bio-Rad Laboratories, CA, USA) using the ChemiDoc instrument (Bio-Rad Laboratories, CA, USA).

The same membranes were stripped and reprobed with anti-GAPDH monoclonal antibody (Santa Cruz Biotechnology). Images were quantified by Image J Software (http://rsb.info.nih.gov/ij). C/EBP-β expression in each sample was normalized by dividing the intensity of C/EBP-β bands with GAPDH bands.

The ratio was calculated by dividing the normalized values of stimulated and/or transfected cells by the values of control cells.

### Experiment 6

To test the hypothesis that miR-369-3p upregulation inhibits the production of inflammatory cytokines such as TNF-α and IL-6, dendritic cells preliminarily stimulated with LPS were transiently transfected with miR-369-3p mimic molecules at a concentration of 50 nM.

TNF-α and IL-6 genes expression were significantly reduced after transfection compared to control (mock) (p<0.05, Figure 6A-B).

These results were further confirmed by ELISA data on the supernatant obtained from LPS-stimulated BMDCs after miRNA mimic transfection.

After transfection, the expression of miR-369-3p was elevated and the release of TNF-α and IL-6 was significantly decreased (p <0.05, Figure 6C-D). TNF-α and IL-6 levels in the supernatants were determined using an ELISA kit (R&D Systems, Minneapolis, MN, USA) following the manufacturer's instructions.

Altogether, the results of the inventors show that miR-369-3p upregulation, after exposure to quercetin, is responsible for the reduction of C / EBP-β and therefore of the production of TNF-α and IL-6.

### Experiment 7

This experiment was carried out in order to verify if the exposure to quercetin could influence the expression of miR-369-3p also at the gastrointestinal level which is the anatomical site most involved in nutritional exposure. Therefore, the expression levels of miR-369-3p in the murine intestine of mice treated with quercetin orally were investigated.

Quercetin was orally administered [0.5 µM/g] every 48h for a week, then on day 7 the intestinal tract was explanted and miR-369-3p expression levels were measured in the duodenum, jejunum, ileum and colon.

Following quercetin administration, an increasing expression of miR-369-3p in the ileum and colon was detected, whereas in the duodenum and jejunum the miR-369-3p expression was similar to that in untreated samples. Of note, only in the colon was a significantly increased expression detected (p < 0.05, Figure 7A).

Furthermore, it has been investigated the effect of oral administration of quercetin in DSS-induced experimental colitis mice models. miR-369-3p expression in colon tissue from mice pretreated with quercetin for 8 days and treated with 2% DSS and quercetin for 10 days showed a significant increase (1.8 fold-increase, p< 0.01) compared to DSS untreated control mice (Figure 7B).

Quercetin [0.5 µM/g] or vehicle (1% sucrose) was orally administered at day 0, 3, 5 and 7. At day 8 mice were sacrificed, the duodenum, jejunum, ileum and colon were explanted and gently washed to remove fecal material. Intestinal segments were cut longitudinally and washed 3 times with cold PBS and 2.5mM EDTA to remove epithelial cells. Starting from day 10, acute colitis was induced by administration of 2% DSS (MP Biomedicals, LLC, USA) in drinking water for 10 days with or without quercetin treatment. At day 20 mice were sacrificed, and the colon extracted for the analysis. Total RNA extraction from tissues was performed with the miRNeasy Mini kit (Qiagen) according to the manufacturer's protocol.

The investigation has been conducted in accordance with the ethical standards and according to national and international guidelines and has been approved by the authors' institutional review board (Organism for Animal Wellbeing - OPBA).

Six-to eight-week-old C57BL/6 mice (Stock No: 000664; weight: approximately 20gr) were purchased from Jackson Laboratories. All animal experiments were carried out in accordance with Directive 86/609 CEE enforced by Italian D.L. n. 26/2014 and approved by the Committee on the Ethics of Animal Experiments of Ministero della Salute - Direzione Generale Sanità Animale (Prot. n. 768/2015-PR 27/07/2015) and the official RBM veterinarian.

### Experiment 8

This experiment allowed to determine the expression levels of miR-369-3p in intestinal tissue human patients with chronic inflammatory bowel disease (IBD); therefore, RNA samples extracted from intestinal inflamed regions compared with the non-inflamed regions obtained by the same patients have been analyzed.

As shown in Figure 8, miR-369-3p in the inflamed areas was significantly lower than those in non-inflamed tissue (1.85-fold decrease, p <0.001). This result confirms the involvement of miR-369-3p in the regulation of the inflammatory cascade, specifically in inflammatory bowel disease.

Intestinal samples from non-inflamed and inflamed regions were obtained from 11 IBD patients recruited in the Department of Gastroenterology of the National Institute of Gastroenterology "S. de Bellis". Written informed consent was obtained from all study participants. The study was carried out according to the principles of the Declaration of Helsinki and was approved by the local Institutional Ethics Review Boards. Total RNA extraction from tissues was performed with the miRNeasy Mini kit (Qiagen) according to the manufacturer's protocol.

### CYTED BIBLIOGRAPHY

Baltimore D., Boldin M.P., O'Connell R.M., Rao D.S., and Taganov K.D. 2008. MicroRNAs: new regulators of immune cell development and function. Nat Immunol. 9:839-845.
Caux C., Vanbervliet B., Massacrier C., Azuma M., Okumura K., Lanier L.L., and Banchereau J. 1994. B70/B7-2 is identical to CD86 and is the major functional ligand for CD28 expressed on human dendritic cells. J. Exp. Med. 180:1841-1847.
Cavalcanti E., Vadrucci E., Delvecchio F.R., Addabbo F., Bettini S., Liou R., Monsurrò V., Huang A.Y., Pizarro T.T., Santino A., and Chieppa M. 2014. Administration of reconstituted polyphenol oil bodies efficiently suppresses dendritic cell inflammatory pathways and acute intestinal inflammation. PLoS One. 9:e88898.
Delvecchio F.R., Vadrucci E., Cavalcanti E., De Santis S., Kunde D., Vacca M., Myers J., Allen F., Bianco G., Huang A.Y., Monsurro V., Santino A., and Chieppa M. 2015. Polyphenol administration impairs T-cell proliferation by imprinting a distinct dendritic cell maturational profile. Eur. J. Immunol. 45:2638-2649.
De Santis S., Kunde D., Serino G., Galleggiante V., Caruso M.L., Mastronardi M., Cavalcanti E., Ranson N., Pinto A., Campiglia P., Santino A., Eri R., and Chieppa M. 2016. Secretory leukoprotease inhibitor is required for efficient quercetin-mediated suppression of TNFa secretion. Oncotarget. 7:75800-75809.
De Santis S., Galleggiante V., Scandiffio L., Liso M., Sommella E., Sobolewski A., Spilotro V., Pinto A., Campiglia P., Serino G., Santino A., Notarnicola M., and Chieppa M. 2017. Secretory Leukoprotease Inhibitor (Slpi) Expression Is Required for Educating Murine Dendritic Cells Inflammatory Response Following Quercetin Exposure. Nutrients. 9: E706.
He L., and Hannon G.J. 2004. MicroRNAs: small RNAs with a big role in gene regulation. Nat Rev Genet. 5:522-531.
Huang R.Y., Yu Y.L., Cheng W.C., OuYang C.N., Fu E., and Chu C.L. 2010. Immunosuppressive effect of quercetin on dendritic cell activation and function. J Immunol. 184:6815-6821.
Hungness E.S., Robb B.W., Luo G.J., Pritts T.A., Hershko D.D., and Hasselgren P.O. 2002 Proteasome inhibitors activate the transcription factors C/EBP-beta and delta in human intestinal epithelial cells. Biochem Biophys Res Commun. 290:469-474.
Inaba K., Turley S., lyoda T., Yamaide F., Shimoyama S., Reis e Sousa C., Germain R.N., Mellman I., and Steinman R.M. 2000. The formation of immunogenic major histocompatibility complex class II-peptide ligands in lysosomal compartments of dendritic cells is regulated by inflammatory stimuli. J Exp Med. 191:927-936.
Janeway C.A. Jr, and Medzhitov R. 2002. Innate immune recognition. Annu Rev Immunol. 20:197-216.
Kempuraj D., Madhappan B., Christodoulou S., Boucher W., Cao J., Papadopoulou N., Cetrulo C.L., and Theoharides T.C. 2005. Flavonols inhibit proinflammatory mediator release, intracellular calcium ion levels and protein kinase C theta phosphorylation in human mast cells. Br J Pharmacol. 145:934-944.
Li Y., Yao J., Han C., Yang J., Chaudhry M.T., Wang S., Liu H. and Yin Y. 2016.. Quercetin, Inflammation and Immunity. Nutrients. 8: 167.
Manjeet K.R., and Ghosh B. 1999. Quercetin inhibits LPS-induced nitric oxide and tumor necrosis factor-alpha production in murine macrophages. Int. J. Immunopharmocol. 21:435-443.
Mellman I. 2013. Dendritic cells: master regulators of the immune response. Cancer Immunol Res. 1:145-149.
Nahid M.A., Yao B., Dominguez-Gutierrez P.R., Kesavalu L., Satoh M., and Chan E.K. 2013. Regulation of TLR2-mediated tolerance and cross-tolerance through IRAK4 modulation by miR-132 and miR-212. J Immunol. 190: 1250-1263.
Natsuka S., Akira S., Nishio Y., Hashimoto S., Sugita T., Isshiki H. and Kishimoto T. 1992. Macrophage differentiation-specific expression of NF-IL6, a transcription factor for interleukin-6. Blood. 79:460-466.
Niehof M., Streetz K., Rakemann T., Bischoff S.C., Manns M.P., Horn F., and Trautwein C. 2001. Interleukin-6-induced tethering of STAT3 to the LAP/C/EBPbeta promoter suggests a new mechanism of transcriptional regulation by STAT3. J Biol Chem 276:9016-9027.
O'Connell R.M., Chaudhuri A.A., Rao D.S., and Baltimore D. 2009. Inositol phosphatase SHIP1 is a primary target of miR-155. Proc Natl Acad Sci U S A. 106:7113-7118.
O'Connell R.M., Rao D.S., Chaudhuri A.A., and Baltimore D. 2010. Physiological and pathological roles for microRNAs in the immune system. Nat Rev Immunol. 10:111-122. Palm N.W., and Medzhitov R. 2009. Pattern recognition receptors and control of adaptive immunity. Immunological Reviews. 227: 221-233.
Poli V. 1998. The role of C/EBP isoforms in the control of inflammatory and native immunity functions. The Journal of Biological Chemistry 273:29279-29282.
Rueda A., Barturen G., Lebrón R., Gómez-Martín C., Alganza A., Oliver J.L., and Hackenberg M. 2015. sRNAtoolbox: an integrated collection of small RNA research tools. Nucleic Acids Res. 43: W467-W473.
Scott L.M., Civin C.I., Rorth P. and Friedman A.D. 1992. A novel temporal expression pattern of three C/EBP family members in differentiating myelomonocytic cells. Blood. 80: 1725-1735.
Shimizu M., Li J., Inoue J., and Sato R. 2015. Quercetin represses apolipoprotein B expression by inhibiting the transcriptional activity of C/EBPβ. PLoS One. 10:e0121784.
Smyth L.A., Boardman D.A., Tung S.L., Lechler R., and Lombardi G. 2015. MicroRNAs affect dendritic cell function and phenotype. Immunology. 144:197-205.
Sonkoly E., and Pivarcsi A. 2009. microRNAs in inflammation. Int Rev Immunol. 28:535-561.
Sozzani S., Allavena P., D'Amico G., Luini W., Bianchi G., Kataura M., Imai T., Yoshie O., Bonecchi R., and Mantovani A. 1998. Differential regulation of chemokine receptors during dendritic cell maturation: a model for their trafficking properties. J Immunol. 161:1083-1086.
Stagg A.J., Hart A.L., Knight S.C. and Kamm M.A. 2003. The dendritic cell: its role in intestinal inflammation and relationship with gut bacteria. Gut. 52: 1522-1529. Steinman R.M., and Banchereau J. 2007. Taking dendritic cells into medicine. Nature. 449:419-426.
Taganov K.D., Boldin M.P., Chang, K.J. and Baltimore D. 2006. NF-kappaB-dependent induction of microRNA miR-146, an inhibitor targeted to signaling proteins of innate immune responses. Proc Natl Acad Sci U S A. 103:12481-12486.
Takeda K., Kaisho T., and Akira S. 2003. Toll-like receptors. Annu Rev Immunol 21: 335-376.
Takiguchi M. 1998. The C/EBP family of transcription factors in the liver and other organs. Int J Exp Pathol. 79:369-391.
Trautwein C., Caelles C., Van der Geer P., Hunter T., Karin M., and Chojkier M. 1993. Transactivation by NF-IL6/LAP is enhanced by phosphorylation of its activation domain. Nature 364:544-547.

## Claims

1. A pharmaceutical composition comprising one or more microRna, at least one pharmaceutically acceptable vehicle and/or diluent and/or excipient, **characterized by** comprising miRNA-369-3p as active ingredient.

2. The pharmaceutical composition according to claim 1, **characterized by** reducing the activity of C/EBP-β gene and reducing the production of TNFα e IL-6.

3. The pharmaceutical composition according to claims 1 and 2, **characterized by** affecting the activity of dendritic cells.

4. The pharmaceutical composition according to any one of the preceding claims, **characterized in that** is in liquid, powdered, dried, lyophillized form.

5. The pharmaceutical composition according to any one of the preceding claims, for administration as medicament, in solid form such as tablets, pills, hard capsules, powders, granules, suppository medicines, in semi-solid form such as gel, ointments, lubricants, pastes, in liquid form such as beverages, syrups, vials, drops, collars.

6. The pharmaceutical composition according to claim 5, for use in the treatment of the inflammatory chronic disease.

7. The pharmaceutical composition according to claim 6, for use in the treatment of bowel inflammatory chronic disease.

8. The pharmaceutical composition according to any one of the preceding claims, for use in the treatment of ulcerative colitis, Chron disease, colon cancer associated with colitis, rheumatoid arthritis, dermatitis and psoriasis.

9. The pharmaceutical composition according to claims from 5 to 8, for administration to human and animal beings.

10. The pharmaceutical composition according to any one of the preceding claims from 1 to 9, for administration in association with at least an active ingredient known in the treatment of inflammatory chronic diseases.

11. MiRNA-369-3p for use in the treatment of disorders related to inflammation mediated by innate immunity cells, preferably dendritic cells.

12. MiRNA-369-3p according to claim 11 for use in the treatment of inflammatory chronic diseases.
